# EUROPEAN PATENT APPLICATION

(11) **EP 3 421 000 A1**
(43) Date of publication of application: **02.01.2019**
(21) Application number: 18180393.3
(22) Date of filing: 28.06.2018
(51) Int. Cl.: A61B 18/14, A61B 18/04, A61B 18/22, A61N 7/00

(54) **SYSTEMS FOR ABLATIVE TREATMENT OF IRRITABLE BOWEL DISEASE**

(30) Priority: 29.06.2017 US 201715636737; 29.06.2017 US 201715636714
(71) Applicant: Covidien LP, Mansfield, MA 02048 (US)
(72) Inventor: DAVISON, Terry S., Redwood City, CA California 94061 (US); MAGUIRE, Mark A., Hillborough, CA California 94010 (US); HUSZAR, Hillary K., Redwood City, CA California 94065 (US)
(74) Representative: Maschio & Soames IP Ltd

(57) **Abstract**

Methods, systems, and devices are described for treating diseases of the gastrointestinal tract, including ulcerative colitis, by delivering ablative energy to a tissue surface of the gastrointestinal tract and ablating the tissue to a controlled depth. Ablating the tissue may include removing a biofilm layer or coagulating tissue.

## Description

### BACKGROUND

Inflammatory bowel disease (IBD) generally refers to a condition of chronic inflammation in the human digestive tract and encompasses a variety of specific conditions, including ulcerative colitis and Crohn's disease. Ulcerative colitis is characterized by reoccurring ulcers affecting the colon and rectum, which can cause diarrhea, bleeding, and various levels of abdominal pain.

The etiology of ulcerative colitis is not well understood, and many alternative theories have been proposed, including genetic susceptibility, autoimmune disorders, and environmental influences. A common characteristic in ulcerative colitis is the presence of a biofilm that can cover the epithelial cells and invade crypts within the mucosa. Biofilms appear to have an exopolysaccharide (EPS) matrix that provides a protective barrier for underlying bacteria, thereby reducing the effectiveness of antimicrobial medications or the patient's immune system.

The symptoms of ulcerative colitis are typically treated with pharmaceuticals (e.g., antiinflammatory drugs, biologics, steroids) that attempt to reduce inflammation and suppress the body's immune response. These medications, however, do not address the root cause of ulcerative colitis and therefore do not cure the disease. If a patient is not responding to pharmaceuticals, the effected portions of the colon may be surgically removed (i.e., colectomy). Neither pharmaceuticals nor surgery are desirable treatment options, as neither treat the underlying cause of ulcerative colitis while both are accompanied by significant side effects.

### SUMMARY

The described features generally relate to methods, systems, and devices for treating conditions of the gastrointestinal tract with ablative energy. The techniques described herein may be applied to treat ulcerative colitis, ulcerative proctitis, colitis, irritable bowel disease, Crohn's disease, or dysplastic lesions. In general, energy is applied to tissue within the affected organ (e.g., large bowel or rectum) to ablate some portion of the tissue and/or a biofilm covering the tissue. The ablation may remove the affected (e.g., inflamed or non-viable) portions of the tissue (e.g., the mucosal layer), which may facilitate or stimulate regrowth of normal tissue. The ablation may also remove the biofilm and associated harmful bacteria from the tissue. Also, the ablation may destroy neural abnormalities and arborizing nerve fibers in the mucosa that have developed as a result longstanding chronic inflammation. The described treatment methods may prevent or reverse disease progression, alleviate the symptoms associated the disease, and/or delay or avoid the need for surgical intervention.

The affected tissue may be ablated using several different types of energy, methods, and devices. Examples of ablative energy include radio frequency (RF) energy (direct and saline mediated), gas plasma, laser, and cryotherapy. These different types of energy may be used separately or in combination with each other. Moreover, in addition to ablative energy, antibiotics, probiotics, or other types of pharmaceuticals may be used or applied to treat the affected tissue.

Methods and apparatuses are described for ablative treatment of irritable bowel disease. A method for treating ulcerative colitis in a patient is described. The method may include delivering energy to a tissue surface of a tissue within the large intestine of the patient and ablating the tissue with the delivered energy to a controlled depth.

In some embodiments, ablating the tissue comprises removing a biofilm layer at least partially covering the tissue surface and removing a biofilm layer at least partially penetrating below the tissue surface. In some embodiments, ablating the tissue comprises coagulating tissue within the mucosal layers of the tissue and coagulating tissue below the mucosal layers of the tissue. In some examples, ablating the tissue comprises ablating under conditions selected to initiate regrowth of healthy mucosal tissue. Additionally, ablating the tissue comprises defuntionalizing degenerated arborized nerve fibers within the tissue.

In some embodiments, ablating the tissue to a controlled depth comprises controlling a duration of the delivery of energy, controlling an energy density of the delivered energy, and controlling a temperature of the tissue surface during the delivery of energy.

In some embodiments, delivering energy to the tissue surface comprises delivering energy into crypts within the tissue. In some examples, delivering energy to the tissue surface comprises delivering energy to a fully circumferential portion of the large intestine and delivering energy to a partially circumferential portion of the large intestine. Additionally, delivering energy to the tissue surface may comprise at least partially filling a volume of a section of the large intestine with a plasma.

In some embodiments, the energy comprises radiofrequency energy, bipolar radiofrequency energy, monopolar radiofrequency energy, saline-mediated plasma radiofrequency energy, or a combination thereof. In some embodiments, the energy comprises ultrasonic energy. In some cases, the energy comprises a thermal plasma. The thermal plasma may include argon plasma. In some embodiments, the energy comprises a non-thermal plasma. The non-thermal plasma may include non-thermal dielectric-barrier discharge plasma and saline-mediated plasma. In some embodiments, the energy may include laser energy or cryothermal energy. The method may further include delivering a pharmaceutical substance to the tissue surface.

An apparatus for treating ulcerative colitis in a patient may include an energy delivery element configured to deliver energy to a tissue surface of a tissue within the large intestine of the patient. The apparatus for treating ulcerative colitis in a patient may also include a controller configured to control ablation of the tissue with the delivered energy to a controlled depth. In certain examples, the controlled depth comprises a biofilm layer at least partially covering the tissue surface and a biofilm layer at least partially penetrating below the tissue surface. In certain instances, the controlled depth comprises tissue within the mucosal layers of the tissue and tissue below the mucosal layers of the tissue.

In some embodiments, the controller is configured to control a duration of the delivery of energy, an energy density of the delivery of energy, and a temperature at the tissue surface during the delivery of energy

According to various embodiments, an energy delivery element is provided. The energy delivery element may be configured to delivery energy into crypts within the tissue. The energy delivery element may also be configured to deliver energy to a fully circumferential portion of the large intestine or a partially circumferential portion of the large intestine. Additionally, the energy delivery element may be configured to at least partially fill a volume of a section of the large intestine with a plasma.

In some embodiments, the apparatus for treating ulcerative colitis in a patient may include at least one inflatable member configured to seal off at least one end of the section of the large intestine containing the plasma. In certain examples, the energy delivery element may include a radiofrequency energy delivery element, a bipolar radiofrequency energy delivery element, a monopolar radiofrequency energy delivery element, a saline-mediated plasma radiofrequency energy delivery element or a combination thereof.

In some embodiments, the energy delivery element may include an ultrasonic energy delivery element. In some examples, the energy delivery element may include a thermal plasma energy delivery element. The thermal plasma energy delivery element may include an argon plasma coagulation device. In some embodiments, the energy delivery element may include a non-thermal plasma energy delivery element. The non-thermal plasma energy delivery element may include a non-thermal dielectric-barrier discharge plasma energy delivery element or a saline-mediated plasma energy delivery element. In some embodiments, the energy delivery element may include a laser or a cryothermal energy delivery element. In some embodiments, the apparatus for treating ulcerative colitis in a patient may include a pharmaceutical substance delivery element.

Certain embodiments of the present disclosure may include some, all, or none of the above advantages or features. One or more other technical advantages or features may be readily apparent to those skilled in the art from the figures, descriptions, and claims included herein. Moreover, while specific advantages or features have been enumerated above, various embodiments may include all, some, or none of the enumerated advantages or features.

Further scope of the applicability of the described methods and apparatuses will become apparent from the following detailed description, claims, and drawings. The detailed description and specific examples are given by way of illustration only, since various changes and modifications within the spirit and scope of the description will become apparent to those skilled in the art.

### BRIEF DESCRIPTION OF THE DRAWINGS

FIG. 1 illustrates a system for delivering treatment to a target area within a body lumen in accordance with aspects of the present disclosure.
FIG. 2 illustrates a cross-sectional view of tissue affected by ulcerative colitis in accordance with aspects of the present disclosure.
FIG. 3A illustrates a system including an inflatable expansion member positioned within a body lumen for providing treatment of ulcerative colitis in accordance with aspects of the present disclosure.
FIG. 3B illustrates a side view of the system illustrated in FIG. 3A positioned against a tissue surface in accordance with aspects of the present disclosure.
FIG. 4A illustrates a system including an inflatable expansion member positioned within a body lumen for providing treatment of ulcerative colitis in accordance with aspects of the present disclosure.
FIG. 4B illustrates a side view of the system illustrated in FIG. 4A positioned against a tissue surface in accordance with aspects of the present disclosure.
FIG. 5A illustrates a system including an expandable member positioned within a body lumen for providing treatment of ulcerative colitis in accordance with aspects of the present disclosure.
FIG. 5B illustrates a side view of the system illustrated in FIG. 5A positioned against a tissue surface in accordance with aspects of the present disclosure.
FIG. 6A illustrates a system including a pivotable member positioned within a body lumen for providing treatment of ulcerative colitis in accordance with aspects of the present disclosure.
FIG. 6B illustrates a side view of the system illustrated in FIG. 6A positioned against a tissue surface in accordance with aspects of the present disclosure.
FIG. 7A illustrates a system including dual inflatable expansion members positioned within a body lumen for providing treatment of ulcerative colitis in accordance with aspects of the present disclosure.
FIG. 7B illustrates a side view of the system illustrated in FIG. 7A positioned against a tissue surface in accordance with aspects of the present disclosure.
FIG. 8A illustrates a system positioned within a body lumen for providing treatment of ulcerative colitis in accordance with aspects of the present disclosure.
FIG. 8B illustrates a side view of the system illustrated in FIG. 8A positioned against a tissue surface in accordance with aspects of the present disclosure.
FIGs. 9-10 illustrate a flowcharts of methods for treating ulcerative colitis in accordance with various aspects of the present disclosure.

### DETAILED DESCRIPTION

Methods, systems, and devices are described for treating conditions of the gastrointestinal tract with ablative energy. Several different ablation modalities are described and examples of devices and methods for applying the different ablative energy types are provided. The described examples relate to the treatment of ulcerative colitis, but the described techniques may also be used to treat ulcerative proctitis, colitis, irritable bowel disease, Crohn's disease, dysplastic lesions or any other inflammatory condition of the gastrointestinal tract.

Techniques for treating ulcerative colitis may include delivering energy to the affected tissue and ablating the tissue to a controlled depth. Ablating to a controlled depth may include ablating a biofilm layer covering the tissue surface, ablating one or more specific layers of the affected tissue, ablating within crypts of the mucosal layers of the tissue, and/or ablating nerve fibers in and below the mucosal layers of the tissue. To ablate tissue to a controlled depth, the duration of energy delivery, energy density, and/or temperature of the tissue surface may be monitored and controlled.

Several different ablation modalities may be used to ablate affected tissue. Examples of the types of energy that may be used include radiofrequency (RF) energy, ultrasound energy, laser energy, or cryothermal energy. Radio frequency energy may be applied in either bipolar or monopolar mode. Different types of ablation devices may be used to deliver the ablative energy either focally or more globally within a particular target area. Ablation techniques may include direct contact between the ablation device and the target tissue or by indirect contact between ablative gas or saline to create a plasma and the target tissue. The ablation device used to deliver the ablative energy may be coupled to a generator or some other energy source.

An ablation device may include features to control the contact area between the device and the affected tissue. As described with reference to various figures below, the shape and size of the devices may be tailored to achieve certain favorable characteristics such as a certain RF electrode surface area, controlled depth of removal of the mucosal tissue, or optimal angle for contact with the affected area. Described RF devices may use a bipolar configuration with an alternative array of positive and negative electrodes to perform ablation in the form of coagulation.

Plasma may be used to coagulate, cauterize, or otherwise treat tissue through direct application of a high-energy plasma. In particular, kinetic energy transfer from the plasma to the tissue causes healing, and thus, affects thermal coagulation of bleeding tissue. Techniques for plasma coagulation may utilize a handheld electrosurgical instrument having one or more electrodes energizable by a radio-frequency/electrosurgical generator, which outputs a high-intensity electric field suitable for forming plasma using ionizable media (e.g., saline, inert gas).

In some cases, electrical energy is delivered to a treatment device by way of a bipolar plasma catheter that is sized to fit within a working channel of a flexible endoscope and may be employed, for example, in gastrointestinal procedures. Electrosurgical energy may be provided by a generator and may form an electric field between the electrodes contained within the instrument. In this configuration, plasma is generated within the instrument and is delivered to the patient as gas, which is pushed out of the instrument. Generated plasma can further be categorized as thermal plasma or non-thermal plasma.

As described herein, plasma may be produced by applying energy to a controlled substance which will induce ionization and create excited, energized particles. Plasma performs ablation techniques in the form of molecular dissociation and some coagulation of the affected tissue. RF plasma may ablate crypts within the mucosal layer of tissue and stimulate healing through the formation of new blood vessels or macrophage-mediated phagocytosis.

In some examples, a pharmaceutical substance may be taken oral by the patient or be applied directly to the tissue surface. For example, a pharmaceutical substance may be transferred or infused to treat the affected tissue. Locally delivered pharmaceuticals may induce pro-inflammatory cytokines to signal an immune response.

Aspects of the disclosure are now described in detail with reference to the drawings. Examples of ablative treatment are initially described with reference to the specific features and layers of the affected tissue and how the ablative energy affects these different features. Different examples of ablation devices and ablative energy types are then described. Aspects of the disclosure are further illustrated by and described with reference to flowcharts that describe methods for performing ablative treatment of gastrointestinal diseases. As used herein, the term "clinician" refers to a doctor, surgeon, nurse, or any other care provider and may include support personnel. The term "proximal" will refer to the portion of the device or component thereof that is closer to the clinician and the term 'distal" will refer to the portion of the device or component thereof that is farther from the clinician.

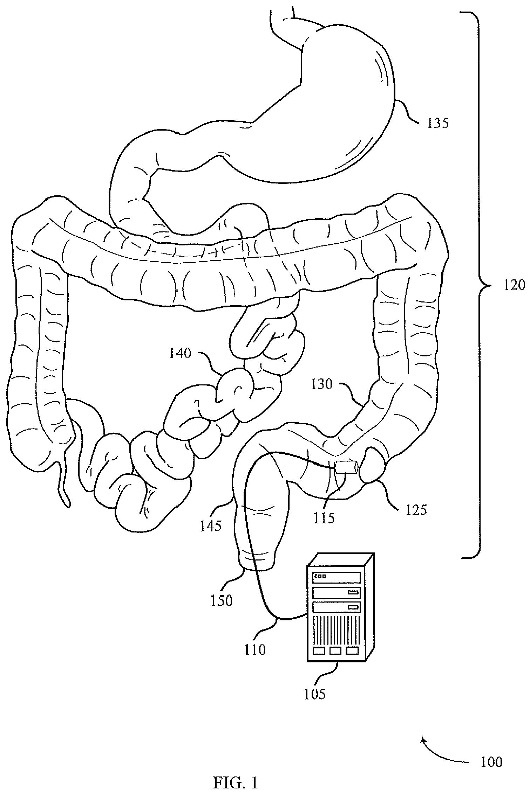

**FIG. 1** shows a system 100 for delivering treatment to a target area within a body lumen in accordance with aspects of the present disclosure. The system 100 may include an energy source 105, a shaft 110, and an energy delivery element 115. The system 100 may be configured to access a body lumen and deliver ablative energy to affected tissue within the lumen to treat ulcerative colitis, for example.

The energy source 105 may be configured to provide ablative energy in a controlled manner to treat affected tissue. In some cases, the ablative energy is provided to ablate tissue to a controlled depth. The energy source 105 may be configured to provide RF energy, ultrasonic energy, plasma, laser energy, or cryothermal energy. Radio frequency energy may be in the form of bipolar RF energy, monopolar RF energy, or saline-mediated plasma RF energy. Plasma RF energy may be thermal (e.g., argon plasma) or non-thermal (e.g., non-thermal dielectric-barrier discharge plasma or saline-mediated plasma).

The energy source 105 may be electrically coupled with the energy delivery element 115 via one or more wires running through the shaft 110. The energy source 105 may also be configured to provide liquid, gas, or plasma to the energy delivery element 115 through one or more lumens running through the shaft 110. The energy source may be configured to monitor parameters associated with the energy delivery and adjust the energy delivery accordingly. For example, the energy source 105 may be configured to monitor the temperature and/or impedance of the tissue being ablated and adjust the level of ablation to maintain a predetermined temperature or impedance level.

The shaft 110 may be configured to support the energy delivery element 115 and provide a means for delivering the energy delivery element 115 to the affected tissue within the body lumen. The shaft 110 may be flexible, steerable, and positioned in the body relative the surface of the affected tissue. In some examples, the shaft 110 may be coupled to an endoscope or may be an endoscope. The shaft 110 may be electrically and/or pneumatically coupled with the energy source 105 to deliver the ablative energy or gas to the energy delivery element 115.

The energy delivery element 115 may be configured to deliver energy from the energy source 105 to affected tissue to ablate or otherwise treat the tissue. For example, the energy delivery element 115 may be configured to deliver RF energy, ultrasonic energy, plasma, laser energy, or cryothermal energy. The energy delivery element 115 may be coupled to a distal end of the shaft 110 and may be configured to pivot or rotate with respect to the shaft 110. In some examples, the energy delivery element 115 is configured to collapse, travel through the body of the shaft 110, and deploy from the distal end of the shaft 110. The energy delivery element 115 may be an expandable or collapsible balloon, a flexible paddle, an arcuate structure, or a combination thereof. Energy delivery element 115 may include apertures configured to accommodate aspiration or secrete liquid or gas to the surface of the affected tissue.

The system 100 may be configured to provide treatment within a body lumen of the gastrointestinal tract 120. For example, the system 100 may be configured to deliver energy to affected tissue 125 within the colon (i.e., large intestine) 130. The affected tissue 125 may be an ulcerated or inflamed area of tissue resulting from ulcerative colitis, for example, or may be a dysplastic lesion. Although the system 100 above is described in the context of treating the colon 130, the system 100 may also be used to treat other organs within the gastrointestinal tract 120 such as the stomach 135, the small intestine 140, the rectum 145 or the anus 150.

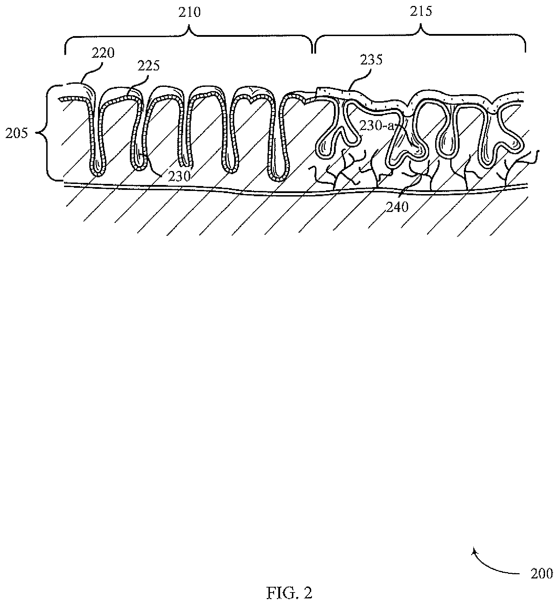

**FIG. 2** illustrates a cross-sectional view of tissue 200 affected by ulcerative colitis in accordance with aspects of the present disclosure. The tissue 200 includes a mucosal layer 205. Tissue 200 includes healthy tissue 210 (e.g., unaffected by gastrointestinal disease) and affected tissue 215 (e.g., affected by a gastrointestinal disease such as ulcerative colitis). The affected tissue 215 may be an example of affected tissue 125 described with reference to FIG. 1. The healthy tissue 210 includes tissue surface 220, goblet cells 225, and crypts 230. Goblet cells 225 may be columnar shaped epithelial cells that line the crypts 230 to secrete mucus to protect the membrane lining. Crypts 230 may be narrow invaginations that extend into the mucosal layer 205.

The affected tissue 215 may be characterized by the presence a biofilm layer 235, the absence of goblet cells 225, the presence of distorted crypts 230-a, and/or the presence of arborized nerve fibers 240. Goblet cells 225 may be absent in the lining of the distorted crypts 230-a due to the interference of goblet cell differentiation caused by inflammatory diseases such as ulcerative colitis. Inflammation associated with ulcerative colitis may distort or abscess the crypts 230-a in the affected tissue 215. Arborized nerve fibers 240 may extend into the mucosal layer 205 as a result of the initial damage to mucosal nerves and resulting over abundant regeneration. In some cases, coagulation of mucosal layers 205 may occur within the mucosal layers 205 or below the mucosal layers 205 of the affected tissue 215.

The biofilm layer 235 may play a role in the pathogenesis of various gastrointestinal diseases such as inflammatory bowel disease (IBD) or ulcerative colitis, leading to the development of ulcers and fissures. The biofilm layer 235 has an exopolysaccharide (EPS) matrix that can act as a protective barrier for potentially detrimental bacteria, shielding it from antimicrobial medication and the body's immune system. The biofilm layer 235 may cause the body's immune system to produce a number of inflammatory cytokines such as IL-1, IFNγ and TNF-α. The coupling of the biofilm layer 235 and the pro-inflammatory response may cause the inflammatory condition to become chronic. The biofilm layer 235 may partially cover the affected tissue 215 or penetrate below the surface of the affected tissue 215. For example, the biofilm layer 235 may adhere to the tissue surface 220, within the crypts 230-a, or be exposed to the mucosal layer 205.

A localized ablative technique may deliver energy to a fully or partially circumferential portion of the affected tissue 215 lining the colon 130. Ablation of the surface of the affected tissue 215 may remove the mucosal layer 205 or eradicate a biofilm layer 235 covering the mucosal layer 205. Energy delivered into the crypts 230-a within the affected tissue 215 may initiate regrowth of the healthy mucosal layer 205 or inhibit regeneration of neural abnormalities and arborized nerve fibers 240 in the mucosal layer 205 that have developed as a result of long standing chronic inflammation. Ablative therapy may stimulate mucosal restitution, with the potential to return normal function and maintain the protective, tissue surface 220 that forms a barrier between the mucosal layer 205 and the microbiome.

In some examples described herein, ablative techniques may include RF ablation (direct or saline mediated), gas plasma ablation, laser ablation, cryotherapy, antibiotic, probiotic, or other modalities, in exclusively or in combination.

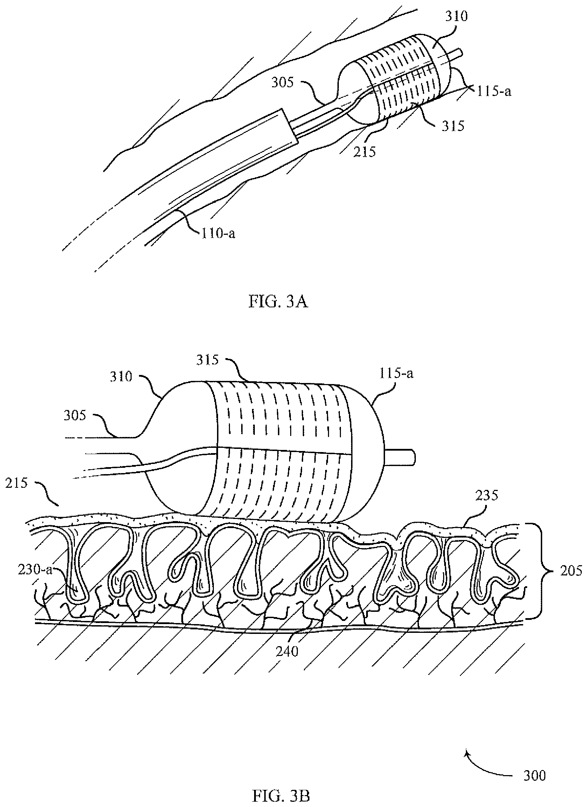

**FIG. 3A** shows a system 300 for providing treatment of ulcerative colitis positioned within a body lumen in accordance with aspects of the present disclosure. The system 300 may include a shaft 110-a, an energy delivery element 115-a, a catheter 305, and an energy source 105 (not shown). The system 300 may be an example of system 100 described with reference to FIG. 1. In accordance with various examples, system 300 may be used to treat ulcerative colitis.

The energy delivery element 115-a may include a catheter 305, an expansion member 310, and an electrode array 315. The expansion member 310 may generally be configured to support the electrode array 315 that may be used to supply therapy in the form of ablative energy to the affected tissue 215. The electrode array 315 may be configured to deliver RF energy, and may include a bipolar configuration (e.g., a bipolar radiofrequency energy delivery element) with alternating array of positive and negative electrodes. The electrode array 315 may be configured to contact a fully circumferential portion of the body lumen.

**FIG. 3B** shows a side view of the system 300 for providing treatment to affected tissue 215 in accordance with aspects of the present disclosure. The system 300 may operate by positioning the shaft 110-a inside a body lumen (e.g., colon 130) and maneuvering the expansion member 310 adjacent to the affected tissue 215. The energy source 105 may then be used to supply energy to the electrode array 315 disposed on the expansion member 310 to ablate the affected tissue 215.

The expansion member 310 may be an inflatable device capable of transitioning between a compressed configuration and an expanded configuration. In some examples, the energy source 105 is configured to inflate the expansion member 310 via a catheter 305 (e.g., with liquid or gas). The collapsed configuration may be generally used when the expansion member 310 is inserted into the body lumen (e.g., colon 130) and when re-positioned therein. When the expansion member 310 is positioned adjacent the affected tissue 215, the expansion member 310 may be expanded, such as by inflating from a deflated state (i.e., the compressed configuration) to a substantially inflated state (i.e., the expanded configuration).

As shown in FIGs. 3A-B, the expansion member 310 may be configured to support an electrode array 315. In some examples, the electrode array 315 is a therapeutic or diagnostic instrument, such as an ablation element that provides ablative energy to the affected tissue 215. The electrode array 315 may be configured to make direct contact with the affected tissue 215 by pressing the electrode array 315 against the affected tissue 215.

The electrode array 315 may be mounted to the expansion member 310 in a variety of ways. The electrode array 315 may be integrated within or mounted/attached to the expansion member 310, for example by etching, mounting, or bonding. In some examples, the electrode array 315 may be mounted to an electrode support that is mounted to the expansion member 310. The electrode support may be non-distensible which may maintain the electrode density of the electrode array 315 even though the surface of the expansion member 310 may vary during expansion. In yet other examples, the electrode array 315 may be arranged on an electrode support which is furled around the expansion member 310 in an overlapping manner, such that the support unfurls as the expansion member 310 expands.

The expansion member 310 may be coupled with the catheter 305 and shaft 110-a such that the expansion member 310 may be maneuvered through a channel of the body, such as the colon 130, and positioned adjacent to the affected tissue 215. The shaft 110-a may include a proximal end and a distal end, with the proximal end configured to be coupled with the energy source 105 and the distal end configured to support or otherwise manipulate the expansion member 310. As shown, the shaft 110-a may include an opening configured to allow the catheter 305 to slidably movable relative to the shaft 110-a. Rotating the distal portion of the shaft 110-a may provide torque to the expansion member 310 (either directly or via the catheter 305) and allow for controlled movement and control of the expansion member 310 relative to the affected tissue 215.

The energy source 105 may generally provide ablative energy to the electrode array 315 disposed on the expansion member 310. In some examples, energy is provided from the energy source 105 to the electrode array 315 via one or more transmission lines extending between the energy source 105 and the expansion member 310 and housed within a channel of the shaft 110-a. The energy source 105 may be a bipolar generator, for example.

The expansion member 310 may be sized to expand to fill a partial or fully circumferential portion of the colon 130. System 300 may perform ablation in the form of coagulation by removing the biofilm layer 235 at least partially covering or penetrating below the surface of the affected tissue 215 or coagulating tissue within or below the mucosal layers 205 of the affected tissue 215. Ablating the affected tissue 215 may include providing ablative energy to a controlled depth of the mucosal layer 205. Ablating the affected tissue 215 to a controlled depth may include controlling duration of energy delivery, energy density of delivered energy, and temperature of the surface of the affected tissue 215. For example, ablative energy emitted from electrode array 315 may enter the crypts 230-a and initiate regrowth of the healthy mucosal layer 205 or may defunctionalize degenerated arborized nerve fibers 240 within the affected tissue 215.

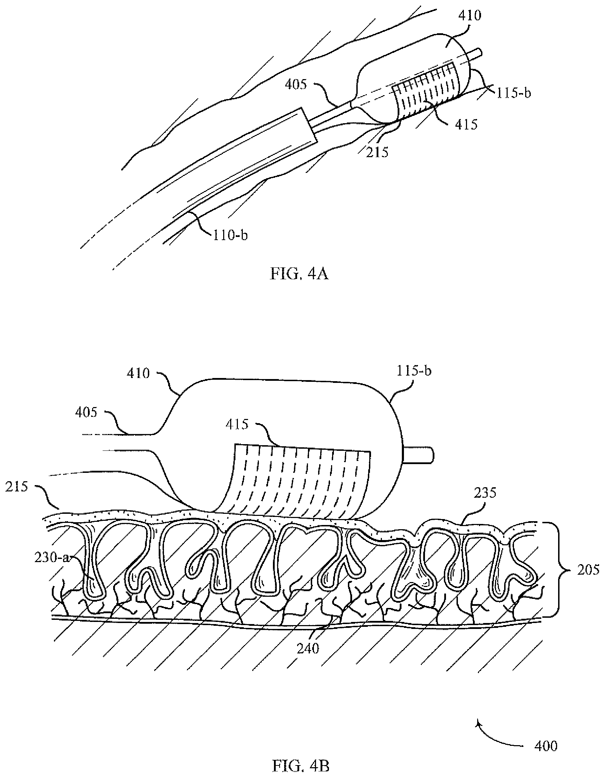

**FIG. 4A** shows a system 400 for providing treatment of ulcerative colitis positioned within a body lumen in accordance with aspects of the present disclosure. The system 400 includes a shaft 110-b, an energy delivery element 115-b, and an energy source 105 (not shown). The system 400 may be an example of system 100 described with reference to FIG. 1. In accordance with various embodiments, system 400 may be used to treat ulcerative colitis.

The energy delivery element 115-b may include a catheter 405, an expansion member 410, and an electrode array 415. The expansion member 410 may generally be configured to support the electrode array 415 that may be used to supply therapy in the form of ablative energy to the affected tissue 215. The electrode array 415 may be configured to deliver RF energy, and may include a bipolar configuration with alternating array of positive and negative electrodes. The electrode array 415 may be configured to contact a partially circumferential portion of the expansion member 410.

**FIG. 4B** shows a side view of the system 400 for providing treatment to affected tissue 215 in accordance with aspects of the present disclosure. The system 400 may operate by positioning the shaft 110-b inside a body lumen (e.g., colon 130) and maneuvering the expansion member 410 adjacent to the affected tissue 215. The energy source 105 may then be used to supply energy to the electrode array 415 disposed on the expansion member 410 to ablate the affected tissue 215.

The expansion member 410 may be an inflatable device capable of transitioning between a compressed configuration and an expanded configuration with the use of supplementary expansion mechanisms. In some examples, the energy source 105 is configured to inflate the expansion member 410 via catheter 405. The expansion member 410 may be an example of the expansion member 310 described with reference to FIG. 3.

As shown in FIGs. 4A-B, the expansion member 410 may be configured to support an electrode array 415. In some examples, the electrode array 415 is a therapeutic or diagnostic instrument, such as an ablation element that provides ablative energy to the affected tissue 215. The electrode array 415 may be configured to make direct contact with the affected tissue 215 by pressing the electrode array 415 against the affected tissue 215. The electrode array 415 may be an example of the electrode array 315 described with reference to FIG. 3.

The electrode array 415 may be mounted to the expansion member 410 in a variety of ways. The electrode array 415 can be integrated within or mounted/attached to the expansion member 410, for example by etching, mounting, or bonding. In some examples, the electrode array 415 may extend only around a partially circumferential portion of the expansion member 410. A partially circumferential configuration may allow for more localized treatment as compared to a fully circumferential electrode configuration.

The expansion member 410 may be coupled with the catheter 405 and shaft 110-b such that the expansion member 410 may be maneuvered through a channel of the body, such as the colon 130, and at the affected tissue 215. The arrangement of the catheter 405 and shaft 110-b may be similar to the arrangement described with reference to FIG. 3.

The energy source 105 may generally provide ablative energy to the electrode array 415 disposed on the expansion member 410. In some examples, energy is provided from the energy source 105 to the electrode array 415 via one or more transmission lines extending between the energy source 105 and the expansion member 410 and housed within a channel of the shaft 110-b.

The expansion member 410 may be sized to expand to fill a partial or fully circumferential portion of the colon 130. Similar to the system 100 and system 300, system 400 may perform ablation in the form of coagulation by removing the biofilm layer 235 at least partially covering or penetrating below the surface of the affected tissue 215 or coagulating tissue within or below the mucosal layers 205 of the affected tissue 215.

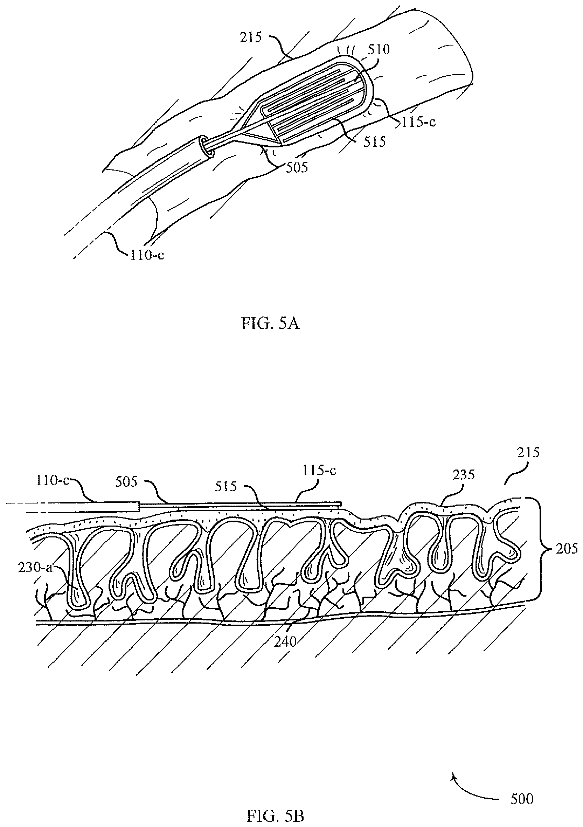

**FIG. 5A** shows a system 500 for providing treatment of ulcerative colitis positioned within a body lumen in accordance with aspects of the present disclosure. The system 500 may include a shaft 110-c, an energy delivery element 115-c, and an energy source 105 (not shown). The system 500 may be an example of system 100 described with reference to FIG. 1. In accordance with various examples, system 500 may be used to treat ulcerative colitis.

The energy delivery element 115-a may include a support member 505, an expansion member 510, and an electrode array 515. The support member 505 may include flexible material (e.g., silicone) that supports the electrode array 515. The expansion member 510 may include one or more members configured to expand and provide structural support to the support member 505. In some examples, the expansion member 510 includes one or more spring-like elements (e.g., nitinol or polymeric strips) that are coupled with the support member 505. The electrode array 515 may be configured to deliver RF energy, and may include a bipolar configuration with alternating array of positive and negative electrodes.

**FIG. 5B** shows a side view of the system 500 for providing treatment to affected tissue 215 in accordance with aspects of the present disclosure. The system 500 may operate by positioning the shaft 110-c inside a body lumen (e.g., colon 130), pushing the support member 505 distally from the distal end of the shaft 110-c, and maneuvering the support member 505 adjacent to the affected tissue 215. The energy source 105 may then be used to supply energy to the electrode array 515 disposed on the support member 505 to ablate the affected tissue 215.

The support member 505 may be a self-expanding device capable of transitioning between a collapsed configuration and an expanded configuration. The support member 505 may be configured to collapse (e.g., roll or fold into a compacted configuration) when inside of the shaft 110-c. When the support member 505 emerges from the shaft 110-c, the support member 505 may self-expand (e.g., unroll, unfold, or otherwise flatten out). As described above, the expansion member 510 may provide structural support to assist in the expansion of the support member 505 upon exiting the shaft 110-c. The support member 505 may also be configured to collapse back into a compacted configuration as it is pulled proximally an retracted back into the shaft 110-c. In the expanded configuration shown in FIG. 5B, the support member 505 may be unfolded into a generally planar surface to contact the affected tissue 215.

As shown in FIGs. 5A-B, the support member 505 may be configured to support an electrode array 515. In some examples, the electrode array 515 is a therapeutic or diagnostic instrument, such as an ablation element that provides ablative energy to the affected tissue 215. The electrode array 515 may be configured to make direct contact with the affected tissue 215 by pressing of the electrode array 515 against the affected tissue 215.

The support member 505 may be configured to support the electrode array 515 in a variety of ways. In some examples, the support member 505 may include a solid elastomeric body on which the electrode array 515 is supported. The support member 505 may thus be a flexible material capable of being curved or folded. The support member 505 may generally have a paddle shape, including a rounded distal end. The support member 505 may taper at the proximal end and couple to the shaft 110-c. As shown in FIG. 5A, the expansion member 510 may include three flexible supports arranged in a "trident" configuration in accordance with various examples. While FIG. 5A shows using from one to three flexible supports, any number of flexible supports can be used. Additionally, the flexible supports can be linear or longitudinal supports.

In some examples, the electrode traces of the electrode array 515 may be aligned parallel with an axis of the support member 505 and may include a backing layer on which the electrodes are disposed. The backing layer, which can include an insulator, may then be disposed on the support member 505. In some examples, the electrodes may be disposed directly on the support member 505. By aligning the electrodes parallel with the axis, the electrode array 515 may be configured to collapse around the axis, as the electrodes will generally not resist the collapsing movement due to their parallel orientation.

The energy source 105 may generally provide ablative energy to the electrode array 515 disposed on the expansion member 510. In some examples, energy is provided from the energy source 105 to the electrode array 515 via one or more transmission lines extending between the energy source 105 and the expansion member 510 and housed within a channel of the shaft 110-c.

The electrode array 515 may emit ablative energy to the affected tissue 215 upon contact. System 500 may perform ablation in the form of coagulation by removing the biofilm layer 235 at least partially covering or penetrating below the surface of the affected tissue 215 or coagulating tissue within or below the mucosal layers 205 of the affected tissue 215. Ablating the affected tissue 215 may include providing ablative energy to a controlled depth of the mucosal layer 205. Ablating the affected tissue 215 to a controlled depth may include controlling duration of energy delivery, energy density of delivered energy, and temperature of the surface of the affected tissue 215. For example, ablative energy emitted from electrode array 515 may enter the crypts 230-a and initiate regrowth of the healthy mucosal layer 205 or defunctionalize degenerated arborized nerve fibers 240 within the affected tissue 215.

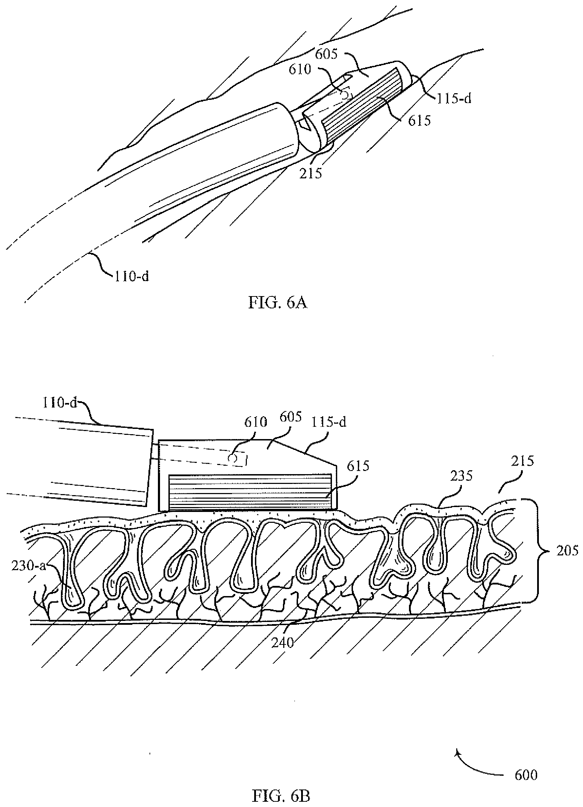

**FIG. 6A** shows a system 600 for providing treatment of ulcerative colitis within a body lumen in accordance with aspects of the present disclosure. The system 600 includes a shaft 110-d, an energy delivery element 115-d, and an energy source 105 (not shown). The system 600 may be an example of system 100 described with reference to FIG. 1. In accordance with various examples, system 600 may be used to treat ulcerative colitis.

The energy delivery element 115-d may include a support member 605, a pin 610, and an electrode array 615. The support member 605 may generally be configured to support the electrode array 615 that may be used to supply therapy in the form of ablative energy to the affected tissue 215. The support member 605 may include an arcuate structure configured to pivot with respect to the shaft 110-d. The electrode array 615 may be configured to deliver RF energy, and may include a bipolar configuration with alternating array of positive and negative electrodes.

**FIG. 6B** shows a side view of the system 500 for providing treatment to affected tissue 215 in accordance with aspects of the present disclosure. The support member 605 may be configured to pivot about the pin 610 so that the support member 605 and the electrode array 615 may contact the surface of the affected tissue 215 regardless of the angle of the shaft 110-d.

The energy source 105 may generally provide energy to the electrode array 615 disposed on the support member 605. In some embodiments, energy is provided from the energy source 105 to the electrode array 615 via conductive wires run through the body of the shaft 110-d to connect the energy delivery element 115-d to an energy source 105. The conductive wires may include a single wire or plurality of wires as needed to provide controlled energy delivery through the electrode array 615 to the affected tissue 215.

The electrode array 615 may emit ablative energy to the affected tissue 215 to treat ulcerative colitis in a variety of ways. System 600 may perform ablation in the form of coagulation by removing the biofilm layer 235 at least partially covering or penetrating below the surface of the affected tissue 215 or coagulating tissue within or below the mucosal layers 205 of the affected tissue 215. Ablating the affected tissue 215 may include providing ablative energy to a controlled depth of the mucosal layer 205. Ablating the affected tissue 215 to a controlled depth may include controlling duration of energy delivery, energy density of delivered energy, and temperature of the surface of the affected tissue 215. For example, ablative energy emitted from electrode array 615 may enter the crypts 230-a and initiate regrowth of the healthy mucosal layer 205 or defunctionalize degenerated arborized nerve fibers 240 within the affected tissue 215.

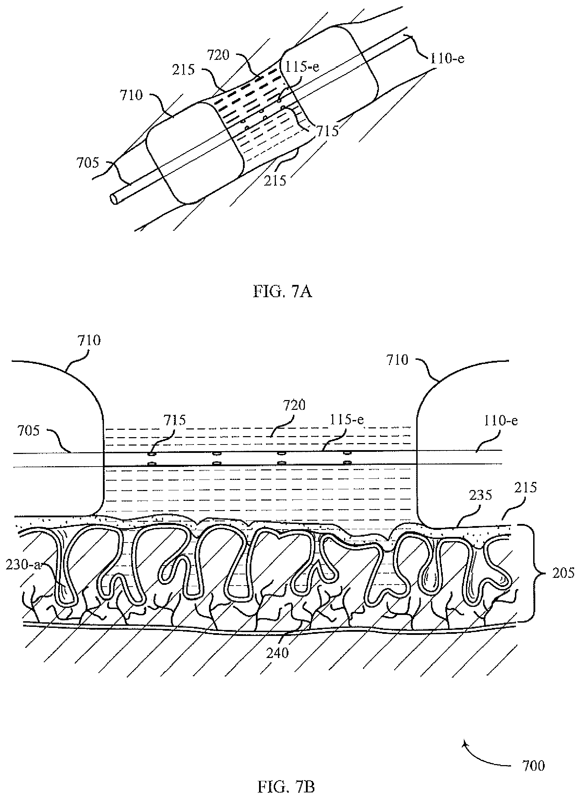

**FIG. 7A** shows a system 700 for providing treatment of ulcerative colitis within a body lumen in accordance with aspects of the present disclosure. The system 700 may include a shaft 110-e, an energy delivery element 115-e, and an energy source 105 (not shown). The system 700 may be an example of system 100 described with reference to FIG. 1. In accordance with various embodiments, system 700 may be used to treat ulcerative colitis.

The energy delivery element 115-e may include a catheter 705, expansion members 710, and apertures 715. The apertures 715 may be configured to secrete an ablative fluid 720 in the form of liquid, gas, or plasma to treat the affected tissue 215.

**FIG. 7B** shows a side view of the system 700 for providing treatment to affected tissue 215 in accordance with aspects of the present disclosure. The system 700 may operate by positioning the shaft 110-e inside a body lumen (e.g., colon 130) and inflating the expansion members 710 until they fully contact the inner circumferential surface of the body lumen, thereby creating a portion of the body lumen between the expansion members 710 that is sealed off from the remaining portions of the body lumen. Then ablative fluid 720 may be delivered through apertures 715 to treat the affected tissue 215 without affecting the other portions of the body lumen. In some examples, the ablative fluid 720 is provided from the energy source 105 to apertures 715 via the catheter 705. Additionally or alternatively, the expansion members 710 may include apertures (e.g., a weeping balloon) through which to deliver ablative fluid 720. In yet other examples, an ablative fluid 720 may be delivered through a porous film or patty that is supported by the catheter 705.

The expansion members 710 may treat ulcerative colitis in a variety of ways. In some examples, the ablative fluid 720 may be a plasma that partially or completely fills a volume of the sealed off section. The ablative fluid 720 may produce a field effect to ablate large sections of the affected tissue 215. The ablative fluid 720 may be saline-mediated RF plasma, which may ablate the affected tissue 215 via a combination of molecular dissociation and coagulation. In some examples, bipolar RF plasma may stimulate healing mediators such as VEGF and HSP-70. The ablative fluid 720 may also penetrate within the crypts 230-a of the tissue. For example, the ablative fluid 720 may enter the crypts 230-a and initiate regrowth of the healthy mucosal layer 205 or defunctionalize degenerated arborized nerve fibers 240 within the affected tissue 215. The ablative fluid 720 may also remove a diseased mucosal layer and/or the biofilm layer 235.

The expansion members 710 may be an inflatable device capable of transitioning between a compressed configuration and an expanded configuration (e.g., a balloon). In some examples, the energy source 105 is configured to inflate the expansion members 710. The collapsed configuration may be generally used when the expansion members 710 are inserted into the lumen and when re-positioned therein. When the expansion members 710 obtains a desired ablation positioning, the expansion members 710 may expand, such as by inflating from a deflated state to a substantially inflated state.

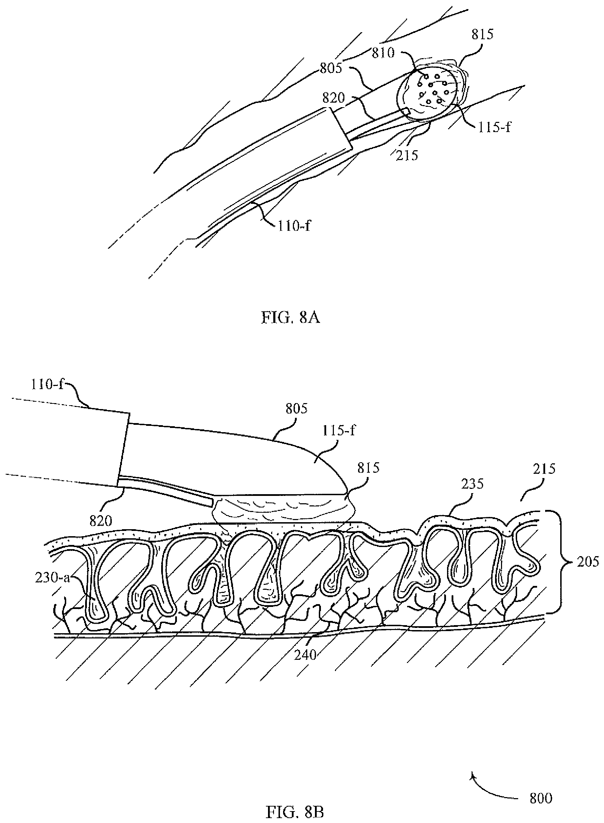

**FIG. 8A** shows a system 800 for providing treatment of ulcerative colitis within a body lumen in accordance with aspects of the present disclosure. The system 800 includes a shaft 110-f, an energy delivery element 115-f, and an energy source 105 (not shown). The energy delivery element 115-f may include a support member 805,apertures 810, and a tube 820. The energy delivery element 115-e may treat the affected tissue 215 by delivering an ablative fluid 815 in the form of liquid, gas, or plasma via the apertures 810. The tube 820 may be coupled with the ablation device to accommodate aspiration. The system 800 may be an example of system 100 described with reference to FIG. 1. In accordance with various embodiments, system 800 may be used to treat ulcerative colitis.

**FIG. 8B** shows a side view of the system 800 for providing treatment to affected tissue 215 in accordance with aspects of the present disclosure. The system 800 may operate by positioning the shaft 110-f inside a body lumen (e.g., colon 130) and maneuvering the support member 805 adjacent to the affected tissue 215. The energy source 105 may be used to supply energy (e.g., ablative fluid 815) through apertures 810 disposed on the support member 805 to treat the affected tissue 215.

For example, the ablative fluid 815 may be in the form of RF plasma such as saline-mediated plasma RF energy and may be an example of a saline-mediated plasma radiofrequency energy delivery element. The plasma RF energy may be in the form of thermal plasma or non-thermal plasma. Non-thermal plasma may include non-thermal dielectric-barrier discharge plasma or saline mediated plasma. In some examples, the energy delivery element 115-e may be configured to deliver RF energy, such as bipolar RF energy or monopolar RF energy. In some cases, RF energy may be applied to a saline solution to create a bipolar plasma. In yet other examples, the energy delivery element 115-e may be configured to deliver ultrasound energy configured to ablate tissue. In some examples, the energy delivery element 115-e may be configured to deliver laser energy or cryothermal energy configured to desiccate and/or vaporize the affected tissue 215, a biofilm layer 235, and/or ulcers of the colon. The energy delivery element 115-e may be coupled with or without aspiration.

System 800 may perform ablation by removing the biofilm layer 235 at least partially covering or penetrating below the surface of the affected tissue 215 or coagulating tissue within or below the mucosal layers 205 of the affected tissue 215. In some examples, the tube 820 may be coupled with the ablation device to aspirate a saline solution and remove ablated by-products of the affected tissue 215. In some examples, the ablative fluid 815 may additionally or alternatively include a pharmaceutical substance or agent (e.g., probiotics, ciprofloxacin and/or pro-inflamatory cytokine-based therapeutics and/or antimicrobial drugs) delivered to the affected tissue 215. The ablative fluid 815 may enter the crypts 230-a and initiate regrowth of the healthy mucosal layer 205 or defunctionalize degenerated arborized nerve fibers 240 within the affected tissue 215.

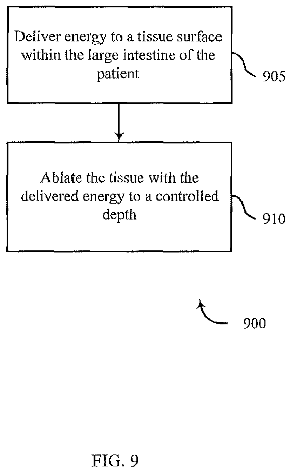

**FIG. 9** shows a flowchart for a method 900 for treating ulcerative colitis in accordance with various aspects of the present disclosure. The steps of method 900 may be performed with any of the systems or components described with reference to FIGs. 1-8 and may be an example of aspects of the particular procedure described with reference to FIGs. 3-8. At block 905, the method 900 may include delivering energy to a tissue surface within the large intestine of the patient. At block 910, the method 900 may further include ablating the tissue with the delivered energy to a controlled depth.

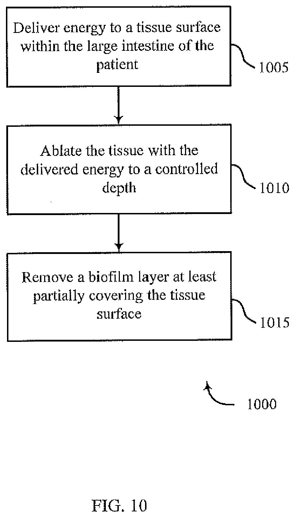

**FIG. 10** shows a flowchart for a method 1000 for treating ulcerative colitis in accordance with various aspects of the present disclosure. The steps of method 1000 may be performed with any of the systems or components described with reference to FIGs. 1-8 and may be an example of aspects of the particular procedure described with reference to FIGs. 3-8. At block 1005, the method 1000 may include delivering energy to a tissue surface within the large intestine of the patient. At block 1010, the method 1000 may further include ablating the tissue with the delivered energy to a controlled depth. At block 1015, the method 1000 may include removing a biofilm layer at least partially covering the tissue surface.

The description herein is provided to enable a person skilled in the art to make or use the disclosure. Various modifications to the disclosure will be readily apparent to those skilled in the art, and the generic principles defined herein may be applied to other variations without departing from the scope of the disclosure. Thus, the disclosure is not limited to the examples and designs described herein, but is to be accorded the broadest scope consistent with the principles and novel features disclosed herein.

The invention may be described by reference to the following numbered paragraphs:-
1. An apparatus for treating ulcerative colitis in a patient, comprising:
   an energy delivery element configured to deliver energy to a tissue surface of a tissue within the large intestine of the patient; and
   a controller configured to control ablation of the tissue with the delivered energy to a controlled depth.
2. The apparatus of paragraph 1, wherein the controlled depth comprises a biofilm layer at least partially covering the tissue surface.
3. The apparatus of paragraph 1, wherein the controlled depth comprises a biofilm layer at least partially penetrating below the tissue surface.
4. The apparatus of paragraph 1, wherein the controlled depth comprises tissue within the mucosal layers of the tissue.
5. The apparatus of paragraph 1, wherein the controlled depth comprises tissue below the mucosal layers of the tissue.
6. The apparatus of paragraph 1, wherein the controller is configured to control a duration of the delivery of energy.
7. The apparatus of paragraph 1, wherein the controller is configured to control an energy density of the delivery of energy.
8. The apparatus of paragraph 1, wherein the controller is configured to control a temperature at the tissue surface during the delivery of energy.
9. The apparatus of paragraph 1, wherein the energy delivery element is configured to delivery energy into crypts within the tissue.
10. The apparatus of paragraph 1, wherein the energy delivery element is configured to deliver energy to a fully circumferential portion of the large intestine.
11. The apparatus of paragraph 1, wherein the energy delivery element is configured to deliver energy to a partially circumferential portion of the large intestine.
12. The apparatus of paragraph 1, wherein the energy delivery element is configured to at least partially fill a volume of a section of the large intestine with a plasma.
13. The apparatus of paragraph 12, further comprising:
   at least one inflatable member configured to seal off at least one end of the section of the large intestine containing the plasma.
14. The apparatus of paragraph 1, wherein the energy delivery element comprises a bipolar radiofrequency energy delivery element, a monopolar radiofrequency energy delivery element, a saline-mediated plasma radiofrequency energy delivery element, or a combination thereof.
15. The apparatus of paragraph 1, wherein the energy delivery element comprises an ultrasonic energy delivery element.
16. The apparatus of paragraph 1, wherein the energy delivery element comprises a thermal plasma energy delivery element.
17. The apparatus of paragraph 1, wherein the energy delivery element comprises a non-thermal plasma energy delivery element.
18. The apparatus of paragraph 1, wherein the energy delivery element comprises a laser.
19. The apparatus of paragraph 1, wherein the energy delivery element comprises a cryothermal energy delivery element.
20. The apparatus of paragraph 1, further comprising:
   a pharmaceutical substance delivery element.

## Claims

1. An apparatus for treating ulcerative colitis in a patient, comprising:
an energy delivery element configured to deliver energy to a tissue surface of a tissue within the large intestine of the patient; and
a controller configured to control ablation of the tissue with the delivered energy to a controlled depth.

2. The apparatus of claim 1, wherein the controlled depth comprises a biofilm layer at least partially covering the tissue surface.

3. The apparatus of claim 1, wherein the controlled depth comprises a biofilm layer at least partially penetrating below the tissue surface.

4. The apparatus of claim 1, wherein the controlled depth comprises tissue within the mucosal layers of the tissue.

5. The apparatus of claim 1, wherein the controlled depth comprises tissue below the mucosal layers of the tissue.

6. The apparatus of any preceding claim, wherein the controller is configured to control at least one of:
a duration of the delivery of energy;
an energy density of the delivery of energy;and
a temperature at the tissue surface during the delivery of energy.

7. The apparatus of any preceding claim, wherein the energy delivery element is configured to deliver energy to at least one of:
crypts within the tissue;
a fully or partially circumferential portion of the large intestine;

8. The apparatus of any preceding claim, wherein the energy delivery element is configured to at least partially fill a volume of a section of the large intestine with a plasma, preferably further comprising:
at least one inflatable member configured to seal off at least one end of the section of the large intestine containing the plasma.

9. The apparatus of any preceding claim, wherein the energy delivery element comprises a bipolar radiofrequency energy delivery element, a monopolar radiofrequency energy delivery element, a saline-mediated plasma radiofrequency energy delivery element, or a combination thereof.

10. The apparatus of claim 1, wherein the energy delivery element comprises at least one of:
an ultrasonic energy delivery element;
a thermal plasma energy delivery element;
a non-thermal plasma energy delivery element;
a laser;and
a cryothermal energy delivery element.

11. The apparatus of any preceding claim, further comprising:
a pharmaceutical substance delivery element.

12. Apparatus as claimed in any preceding claim comprising an energy delivery element comprising a support member (605), a pin (610) and an electrode array (615) wherein the support member is configured to support the electrode array and is pivotably connected by the pin to a shaft.

13. Apparatus as claimed in claim 12 wherein the support member is arcuate.

14. Apparatus as claimed in any one of claims 1 to 11 wherein the energy delivery element (115e) includes a catheter (705) passing through two spaced apart expansion members (710) wherein the catheter is provided with apertures (715) at positions between the expansion members through which, in use, an ablative fluid may be secreted.

15. Apparatus as claimed in claim 14 wherein the apertures are provided as pores in a film or patty that in supported by the catheter (705).
